Europäisches Patentamt

European Patent Office

Office européen des brevets

(19)

(11) Veröffentlichungsnummer: **0 021 211**
**B1**

(12) **EUROPÄISCHE PATENTSCHRIFT**

(45) Veröffentlichungstag der Patentschrift:
14.07.82

(21) Anmeldenummer: 80103188.1

(22) Anmeldetag: 09.06.80

(51) Int. Cl.³: **C 07 C 69/96, C 07 C 68/02**

(54) Verfahren zur Herstellung von Dimethylcarbonat.

(30) Priorität: 22.06.79 DE 2925209

(43) Veröffentlichungstag der Anmeldung:
07.01.81 Patentblatt 81/1

(45) Bekanntmachung des Hinweises auf die Patenterteilung:
14.07.82 Patentblatt 82/28

(84) Benannte Vertragsstaaten:
BE CH DE FR GB IT LI NL

(56) Entgegenhaltungen:
**DE-B-1 210 853**
**DE-C-349 010**
**US-A-2 787 631**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Buysch, Hans-Josef, Dr., Brandenburgerstrasse 28, D-4150 Krefeld 1 (DE)**
Erfinder: **Krimm, Heinrich, Dr., Heyenbaumstrasse 65, D-4150 Krefeld 1 (DE)**
Erfinder: **Böhm, Siegfried, Dr., Silesiusstrasse 80, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von Dimethylcarbonat

Die Erfindung betrifft ein Verfahren zur Herstellung von Dimethylcarbonat durch Umsetzung von Methanol mit Phosgen und/oder Chlorameisensäuremethylester in Gegenwart einer wäßrigen Alkalihydroxidlösung und in Gegenwart von inerten mit Wasser nicht mischbaren organischen Lösungsmitteln.

Aus der US-Patentschrift 2 787 631 ist bekannt, Dimethylcarbonat durch Umsetzung von Phosgen und Methanol in einem großen Überschuß von Chlorameisensäuremethylester bei Rückflußtemperatur darzustellen.

Weiterhin ist aus der französischen Patentschrift 2 163 884 bekannt, Dimethylcarbonat durch kontinuierliche Umsetzung von Chlorameisensäuremethylester mit Methanol in einer mit Raschig-Ringen gefüllten Kolonne unter Rückflußbedingungen herzustellen.

Da bei den oben geschilderten Umsetzungen von Methanol mit Phosgen oder Chlorameisensäuremethylester Chlorwasserstoff freigesetzt wird und dieser in Verbindung mit Methanol äußerst korrosiv wirkt, müssen die Umsetzungen in Apparaturen aus besonders korrosionsbeständigen Materialien durchgeführt werden (s. Kirk-Othmer; Encyclopedia of chemical Technology, sec. ed., Vol. 4, Seite 391 [1964]), was die Wirtschaftlichkeit der Verfahren beeinträchtigt.

Nachteilig ist weiterhin, daß der entweichende Chlorwasserstoff beträchtliche Mengen an Ausgangs- und Endprodukt mitreißt. Um diese Produkte von dem entweichenden Chlorwasserstoff abzutrennen, bedarf es aufwendiger und kostspieliger Trennoperationen, wie z. B. Tiefkühlung des Chlorwasserstoff-Gases und fraktionierte Destillation der organischen Produkte.

Trotz einer solchen aufwendigen Reinigung des Chlorwasserstoff-Gases verbleiben noch Verunreinigungen, so daß das Chlorwasserstoff-Gas wegen der Verunreinigungen für viele andere Zwecke, z. B. Elektrolyse, nicht geeignet ist und durch Alkaliwäsche neutralisiert und beseitigt werden muß.

Daneben initiiert der freigesetzte Chlorwasserstoff in besonderem Maße Nebenreaktionen, wie die Bildung von Methylchlorid, Dimethylester, Wasser und $CO_2$ (vgl. z. B. J. Chem. Soc. 1935, 600 und Kirk-Othmer, Encyclopedia of chemical Technology, sec. ed., Vol. 4, Seite 387 [1964]). Die Bildung solcher Nebenprodukte in z. T. größeren Mengen wurde auch durch eigene Untersuchungen bei der Umsetzung von Methanol mit Phosgen oder Chlorameisensäuremethylester festgestellt.

Man kann zwar durch besondere Maßnahmen, beispielsweise niedrige Temperatur, die Bildung von Nebenprodukten geringfügig einschränken. Es gelingt jedoch nicht, die Nebenreaktionen zu unterdrücken.

Weiterhin ist es erforderlich, das mit organischem Material belastete, aus sicherheitstechnischen Gründen mit Stickstoff verdünnte Abgas zur Vermeidung von Umweltbelastungen zu verbrennen, was einen erheblichen technischen Aufwand verlangt.

Da das durch Umsetzung von Methanol mit Phosgen oder Chlorameisensäuremethylester hergestellte Dimethylcarbonat, wie zuvor beschrieben, mit Chlorwasserstoff, Methylchlorid, Dimethylester, Spuren von Wasser sowie nicht umgesetztem Chlorameisensäuremethylester und Methanol verunreinigt ist, verlangt die Abtrennung all dieser Substanzen von dem Dimethylcarbonat eine aufwendige Reinigungsoperation und sehr wirksame, korrosionsbeständige Destillationskolonnen.

Es wurde nun ein Verfahren zur Herstellung von Dimethylcarbonat durch Umsetzung von Methanol mit Phosgen und/oder Chlorameisensäuremethylester gefunden, das dadurch gekennzeichnet ist, daß man die Umsetzung in Gegenwart einer wäßrigen Alkalihydroxidlösung und in Gegenwart von inerten, mit Wasser nicht mischbaren organischen Lösungsmitteln bei Temperaturen im Bereich von $-20°C$ bis $+40°C$ durchführt.

Als Alkalihydroxide, die nach dem erfindungsgemäßen Verfahren in Form einer wäßrigen Lösung eingesetzt werden können, kommen in Frage:

Lithiumhydroxid, Natriumhydroxid, Kaliumhydroxid, Caesiumhydroxid und Rubidiumhydroxid, bevorzugt: Natrium- und Kaliumhydroxid, besonders bevorzugt: Natriumhydroxid.

Die Alkalihydroxide können einzeln oder im Gemisch untereinander in Form ihrer wäßrigen Lösung in das erfindungsgemäße Verfahren eingesetzt werden.

Die Konzentration der wäßrigen Alkalihydroxidlösung ist für das erfindungsgemäße Verfahren nicht kritisch. Vorteilhafterweise setzt man jedoch höherprozentige wäßrige Alkalihydroxidlösungen ein. Im allgemeinen werden 15 bis 50gew.-%ige, bevorzugt 25 bis 48gew.-%ige, und besonders bevorzugt 35 bis 45gew.-%ige wäßrige Alkalihydroxidlösungen eingesetzt.

Dies hat den Vorteil, daß die Abwassermenge erniedrigt und die Reaktionszeit verkürzt werden kann.

Die Menge an wäßriger Alkalihydroxidlösung entspricht in der Regel der Menge, die der an zu erwartendem Chlorwasserstoff äquivalent ist. Bevorzugt werden pro Mol Phosgen etwa 2 bis 2,6 Mol oder pro Mol Chlorameisensäuremethylester etwa 1 bis 1,3 Mol Alkalihydroxid in wäßriger Lösung eingesetzt, damit die Reaktion rasch quantitativ abläuft.

Als inerte organische Lösungsmittel können solche in das erfindungsgemäße Verfahren eingesetzt werden, die sich nicht mit der wäßrigen Phase vermischen und zudem leicht destillativ von Dimethylcarbonat abzutrennen sind.

Bevorzugt werden als inerte mit Wasser nicht mischbare organische Lösungsmittel eingesetzt: aliphatische und cycloaliphatische Kohlenwasserstoffe mit 1 bis 30, bevorzugt 5 bis 20 Kohlenstoffatomen; aromatische und araliphatische Kohlenwasserstoffe mit 6 bis 30, bevorzugt 6 bis 10 Kohlenstoffatomen; aliphatische und aralphatische Ether mit 5 bis 30, bevorzugt 6 bis 24 Kohlenstoffatome, Amide, Nitrile und Ester von aliphatischen, araliphatischen und aromatischen Carbonsäuren mit 5 bis 30, bevorzugt 6 bis 12 Kohlenstoffatomen.

Die Verbindungen der genannten Stoffklassen können gegebenenfalls ein- oder mehrfach mit Fluor, Chlor, Brom substituiert sein.

Zum Beispiel seien folgende inerte, mit Wasser nicht mischbare organische Lösungsmittel genannt:

Petrolether (Siedepunkt 50–60°C), Pentan, Isooctan, Testbenzin, Paraffinöl, Dekalin, Toluol, Xylol, Cumol, Diisopropylbenzol, Tetralin, Dodecylbenzol, Methylenchlorid, 1,2-Dichlorpropan, n-Octylchlorid, Brombenzol, Chlorbenzol, Dichlorbenzol, Chlortoluol, Chlorxylol, Diisobutylether, Anisol, Anethol, Hexannitril, Benzonitril, Benzylcyanid, Butylester der Dimethyl- u. Trimethylessigsäure.

Bevorzugt seien genannt:

Chlorbenzol, Chlortoluol, Chlorxylol, Brombenzol, Dichlorbenzol, Xylol, Cumol, Diisopropylbenzol.

Die Menge der eingesetzten inerten, mit Wasser nicht mischbaren organischen Lösungsmittel ist nicht kritisch. Bevorzugt wird etwa die 0,5- bis 20fache Gewichtsmenge, bezogen auf Dimethylcarbonat, eingesetzt.

Die Lösungsmittel können allein oder im Gemisch untereinander in das erfindungsgemäße Verfahren eingesetzt werden.

Die Reaktionstemperaturen des erfindungsgemäßen Verfahrens liegen im allgemeinen im Bereich von etwa −20 bis etwa +40°C, vorzugsweise bei −10 bis +35°C, besonders bevorzugt bei −5 bis +30°C.

Die Reaktionskomponenten Methanol, Phosgen oder Chlorameisensäuremethylester werden im allgemeinen im äquivalenten Molverhältnis eingesetzt, wobei selbstverständlich Methanol auch im Überschuß verwendet werden kann.

Zur weiteren Beschleunigung der Reaktion, insbesondere gegen Ende der Umsetzung, können Katalysatoren zugesetzt werden.

Beispielsweise können tertiäre Amine, wie Triethylamin, Pyridin, Dimethylstearylamin, quartäre Ammoniumsalze, wie Tetrabutylammoniumhydroxid, Triethylbenzylammoniumchlorid sowie quartäre Phosphoniumsalze wie Triethylbenzylphosphoniumchlorid und Tributylcyanethylphosphoniumchlorid in das erfindungsgemäße Verfahren eingesetzt werden.

Die Aufarbeitung des erhaltenen Reaktionsgemisches kann z. B. durch Abtrennen der Dimethylcarbonat enthaltenden organischen Phase von der wäßrigen Phase, Behandlung der organischen Phase mit wäßriger Alkalihydroxidlösung und fraktionierter Destillation der organischen Phase zur Gewinnung von reinem Dimethylcarbonat durchgeführt werden.

Die bei der Destillation erhaltenen Vor- und Nachläufe können wieder unmittelbar in die Reaktion zurückgeführt werden.

Es überrascht außerordentlich, daß bei der Behandlung des rohren Dimethylcarbonats mit wäßriger Alkalihydroxidlösung praktisch keine Verseifung des Dimethylcarbonats stattfindet, da, wie eigene Versuche zeigen, bei der Behandlung mit festen Alkalien große Ausbeuteverluste durch Verseifung von Dimethylcarbonat zu verzeichnen sind.

Nach dem erfindungsgemäßen Verfahren wird Dimethylcarbonat in sehr guten Ausbeuten (bis zu 92%) und hohen Reinheiten (>99,5%ig) erhalten.

Das erfindungsgemäße Verfahren kann auf verschiedene Weise technisch durchgeführt werden. Zum Beispiel kann in ein Gemisch aus Methanol, inertem mit Wasser nicht mischbarem organischem Lösungsmittel und wäßriger Alkalihydroxidlösung Phosgen oder Chlorameisensäuremethylester eingeleitet werden. Ferner können Methanol und Phosgen bzw. Chlorameisensäuremethylester getrennt oder nach vorheriger Mischung zu einer wäßrigen Alkalihydroxidlösung zudosiert werden. Es ist auch möglich, die wäßrige Alkalihydroxidlösung einem Gemisch aus Methanol, inertem mit Wasser nicht mischbarem organischem Lösungsmittel und Phosgen bzw. Chlorameisensäuremethylester zuzugeben.

Die Umsetzung wird im allgemeinen bei Temperaturen im Bereich von etwa −20 bis etwa +40°C durchgeführt.

Nach Beendigung der Reaktion wird die organische Phase von der wäßrigen Phase abgetrennt, anschließend getrocknet, beispielsweise mit konzentrierter Alkalihydroxidlösung (etwa 25 bis 50gew.-%ig) behandelt, und nach Abtrennen der Alkalihydroxidlösung fraktioniert destilliert.

Der bei der Destillation erhaltene Vor- und Nachlauf kann wieder unmittelbar in das Reaktionsgemisch zurückgeführt werden.

Die wäßrigen Phasen werden vorteilhafterweise mit inerten, mit Wasser nicht mischbaren organischen Lösungsmitteln behandelt und nach Abtrennen dler organischen Phase zur Abwasseraufbereitung gegeben.

Die inerten organischen Lösungsmittel können wieder für den nächsten Ansatz verwendet werden.

Nach dem erfindungsgemäßen Verfahren wird Dimethylcarbonat in sehr guten Ausbeuten und hohen Reinheiten erhalten. Das Verfahren verläuft außerordentlich selektiv, Nebenreaktionen werden weitgehend unterdrückt.

Da bei dem erfindungsgemäßen Verfahren keine gasförmigen Produkte frei werden, entfällt deren Aufarbeitung oder Vernichtung. Dadurch gestaltet sich das Verfahren besonders wirtschaftlich und umweltfreundlich. Darüber hinaus ist die Aufarbeitung des Rohproduktes, das in hoher Reinheit anfällt, denkbar einfach. Aufwendige und teure Reinigungsschritte sind nicht erforderlich.

Da das erfindungsgemäß erhaltene Dimethylcarbonat keinen Chlorameisensäuremethylester enthält, gestaltet sich die destillative Aufarbeitung besonders einfach. Die Abtrennung von überschüssigem Chlorameisensäuremethylester von Dimethylcarbonat erfordert nämlich nach eigenen Versuchen einen sehr hohen technischen Aufwand.

Daneben war es außerordentlich überraschend, daß Dimethylcarbonat in guten Ausbeuten nach dem erfindungsgemäßen Verfahren erhalten werden konnte. Es war nämlich zu erwarten, daß das reaktive Dimethylcarbonat und der von allen Chlorameisensäureestern hydrolyseanfälligste Chlorameisensäuremethylester unter alkalischen Bedingungen verseift werden würden. Die guten Ausbeuten zeigen jedoch, daß dies praktisch nicht der Fall ist. `

Dimethylcarbonat ist ein wichtiges Zwischenprodukt für die Herstellung von Polycarbonaten sowie für die Synthese von Polyurethanen.

Außerdem ist es ein gutes Lösungsmittel für Polymere mit stark polaren Gruppen, wie Polyacrylnitril und Celluloseester (Ullmanns Enzyklopädie der technischen Chemie, Band 14, 4. Aufl., Seite 592).

Die folgenden Beispiele dienen zur Verdeutlichung des erfindungsgemäßen Verfahren ohne es jedoch auf diese Beispiele zu beschränken.

## Beispiel 1

189 g (2 Mol) Chlorkohlensäuremethylester, 76 g (2,4 Mol) Methanol und 800 g Chlorbenzol werden auf 5°C abgekühlt und unter kräftigem Rühren 182,5 g 45%ige Natronlauge (2,05 Mol) innerhalb $1\frac{1}{2}$ Stunden bei 5°C eingetropft. Nach 10 Minuten liegt der pH bei 8 bis 9. Das Salz wird abgesaugt und mit Chlorbenzol gewaschen. Die organische Schicht wird über wasserfreier Pottasche 45 Minuten getrocknet. Das Filtrat wird an einer 1,15-m-Silberspiegelkolonne fraktioniert destilliert.

| | |
|---|---|
| Vorlauf Sdp. 62 bis 86° | 20 g |
| Hauptlauf Sdp. 87 bis 91° | 128 g $n_D^{20}$ 1,3682 |
| Nachlauf Sdp. 92 bis 130° | 10 g |

Vorlauf und Nachlauf werden in den nächsten Ansatz übernommen. So erhöht sich die Ausbeute an Dimethylcarbonat von zunächst 71% auf 79 bis 81% der Theorie. Die Reinheit beträgt 99,8%.

## Beispiel 2

Vorgelegt werden

| | |
|---|---|
| 945 g (10 Mol) | Chlorameisensäuremethylester |
| 384 g (12 Mol) | Methanol |
| 2 kg | Chlorbenzol |

Bei 5°C werden unter Rühren innerhalb $5\frac{1}{2}$ Stunden 910 g 45%ige NaOH (10,25 Mol) zugetropft. 10 Minuten wird nachgerührt. Der pH-Wert sinkt auf 8 bis 9. Die organische (obere) Schicht wird möglichst vollständig abgehebert, zum Rückstand 1 Liter Wasser gegeben. Nach weitgehender Lösung des Salzes wird die obere Schicht abgetrennt. Beide Chlorbenzolschichten werden durch ein dickes Faltenfilter geklärt. Das Filtrat wird an einer 1,15 m Silberspiegelkolonne mit Glasringen destilliert.

| | | |
|---|---|---|
| 1. | 63,5 bis 70° | 34,2 g |
| 2. | 73 bis 87° | 46,1 g |
| 3. | 87 bis 88° | 20,5 g $n_D^{20}$ = 1,3698 |

# 0 021 211

4. 88 bis 89,5°        707,8 g $n_D^{20}$ = 1,3688 (99,7%ig)
5. 90 bis 94,5°        3,8 g $n_D^{20}$ = 1,3763
6. 95 bis 105°         3,8 g $n_D^{20}$ = 1,3699

Ausbeute (Fraktion 4) 79% der Theorie.
Werden die Fraktionen 1, 2, 3, 5 und 6 dem nächsten Ansatz wieder zugesetzt, so erhöht sich die Ausbeute auf 85 bis 88% der Theorie.

## Beispiel 3

In einem 2-l-Dreihalskolben mit Rührer werden 1200 g Chlorbenzol, 284 g (3,0 Mol) Chlorameisensäuremethylester und 114 g (3,6 Mol) Methanol vorgelegt. Die Lösung wird auf 0 bis 5°C abgekühlt. Unter kräftigem Rühren tropft man innerhalb von 6 Stunden 275 g (3,09 Mol) 45%ige NaOH zu und rührt 30 Minuten nach. Man nimmt die Kühlung weg und gibt 565 g destilliertes Wasser zu. Das während der NaOH-Zugabe ausgefallene Kochsalz geht in Lösung und man erhält zwei wasserklare Phasen. Man rührt 30 Minuten nach, ohne die Temperatur zu kontrollieren und trennt die untere wäßrige Phase ab. Die wäßrige Phase wiegt ca. 980 g, die organische Phase 1450 g.

Die organische Phase wird mit 145 g 45%iger NaOH 10 Minuten gerührt. Die wäßrige Phase wird abgetrennt und zur Trocknung des Extraktionschlorbenzols verwendet. Die organische Phase wird über eine Silberspiegelkolonne fraktioniert destilliert. Vorlauf und Nachlauf werden dem nächsten Versuch zugeschlagen. Das in der Destillationsblase verbleibende Chlorbenzol dient im nächsten Versuch als Extraktionsmittel.

Die wäßrige Phase wird mit 1200 g frischem Chlorbenzol 15 Minuten gerührt und zur Abwasseraufbereitung gegeben. Die Chlorbenzolphase wird als Lösungsmittel im nächsten Ansatz verwendet und vor dem Einsatz mit den oben angegebenen 145 g 45%iger NaOH getrocknet. Die zur Trocknung verwendete NaOH wird im nächsten Ansatz verwendet.

Ausbeute der Rohlösung: ca. 90% der Theorie.

## Beispiel 4

Zu einem Gemisch von

| | |
|---|---|
| 945 g | (10 Mol) Chlorameisensäuremethylester |
| 87 g | Vorlauf aus einem analogen vorausgehenden Ansatz |
| 6 g | Nachlauf und |
| 348 g | (12 Mol) Methanol in |
| 2 kg | o-Dichlorbenzol werden bei 5°C in 5$^{1}/_{2}$ Stunden |
| 910 g | 45%ige Natronlauge (10,25 Mol) unter Rühren zugetropft. |

Nach weiteren 15 Minuten werden 1,2 l Wasser zugegeben, die wäßrige Phase abgetrennt, mit ca. 100 g Dichlorbenzol extrahiert, die organische Phase über ein Cellulosebett klar filtriert und destilliert über eine 1,20 m lange verspiegelte Füllkörperkolonne.

Man erhält einen Vorlauf von 113 g (Kp 63 bis 89°) und eine Hauptfraktion von 765 g (Kp 89 bis 90° $n_D^{20}$ = 1,3688 entsprechend 85% der Theorie an Dimethylcarbonat [99,8%ig]). Der Nachlauf beträgt 5 g (Kp 90 bis 165°).

## Patentansprüche

1. Verfahren zur Herstellung von Dimethylcarbonat durch Umsetzung von Methanol mit Phosgen und/oder Chlorameisensäuremethylester, dadurch gekennzeichnet, daß man die Umsetzung in Gegenwart einer wäßrigen Alkalihydroxidlösung und in Gegenwart von inerten, mit Wasser nicht mischbaren organischen Lösungsmitteln bei Temperaturen im Bereich von −20°C bis +40°C durchführt.

2. Verfahren nach Anspruch 1, dadurch gekennzeichnet, daß man eine 15 bis 50gew.-%ige wäßrige Alkalihydroxidlösung einsetzt.

3. Verfahren nach Anspruch 1 und 2, dadurch gekennzeichnet, daß man pro Mol Phosgen 2 bis 2,6 Mol oder pro Mol Chlorameisensäuremethylester 1 bis 1,3 Mol Alkalihydroxid in wäßriger Lösung einsetzt.

4. Verfahren nach Ansprüchen 1 bis 3, dadurch gekennzeichnet, daß man als Alkalihydroxidlösung eine Natrium- und/oder Kaliumhydroxidlösung einsetzt.

5. Verfahren nach Ansprüchen 1 bis 4, dadurch gekennzeichnet, daß man als inerte mit Wasser nicht mischbare organische Lösungsmittel aliphatische und cycloaliphatische Kohlenwasserstoffe mit 1 bis

5

0 021 211

30 Kohlenstoffatomen, aromatische und araliphatische Kohlenwasserstoffe mit 6 bis 30 Kohlenstoffatomen, aliphatische und araliphatische Ether mit 5 bis 30 Kohlenstoffatomen und/oder Amide, Nitrile und Ester von aliphatischen, araliphatischen und aromatischen Carbonsäuren mit 5 bis 30 Kohlenstoffatomen, die alle gegebenenfalls mit Fluor, Chlor oder Brom substituiert sein können, einsetzt.

6. Verfahren nach Ansprüchen 1 bis 5, dadurch gekennzeichnet, daß man als inerte mit Wasser nicht mischbare organische Lösungsmittel Xylol, Cumol, Chlorbenzol, Chlorxylol, Dichlorbenzol, Diisopropylbenzol, Brombenzol einsetzt.

7. Verfahren nach Ansprüchen 1 bis 6, dadurch gekennzeichnet, daß man die rohe Dimethylcarbonat enthaltende organische Lösung mit 25 bis 50gew.-%iger wäßriger Alkalihydroxidlösung behandelt, die Alkalihydroxidlösung abtrennt und die organische Phase destillativ aufarbeitet.

## Claims

1. Process for the preparation of dimethyl carbonate by reacting methanol with phosgene and/or methyl chloroformate, characterised in that the reaction is carried out in the presence of an aqueous alkali metal hydroxide solution and in the presence of inert, water-immiscible organic solvents, at temperatures in the range from $-20°C$ to $+40°C$.

2. Process according to claim 1, characterised in that a 15 to 50% strength by weight aqueous alkali metal hydroxide solution is used.

3. Process according to claims 1 and 2, characterised in that 2 to 2.6 mols, per mol of phosgene, or 1 to 1.3 mols, per mol of methyl chloroformate, of alkali metal hydroxide, in aqueous solution, are used.

4. Process according to claims 1 to 3, characterised in that a sodium hydroxide solution and/or potassium hydroxide solution is used as the alkali metal hydroxide solution.

5. Process according to claims 1 to 4, characterised in that aliphatic and cycloaliphatic hydrocarbons with 1 to 30 carbon atoms, aromatic and araliphatic hydrocarbons with 6 to 30 carbon atoms, aliphatic and araliphatic ethers with 5 to 30 carbon atoms and/or amides, nitriles and esters of aliphatic, araliphatic and aromatic carboxylic acids with 5 to 30 carbon atoms, all of which compounds can optionally be substituted by fluorine, chlorine or bromine, are used as the inert water-immiscible organic solvents.

6. Process according to claims 1 to 5, characterised in that xylene, cumene, chlorobenzene, chloroxylene, dichlorobenzene, diisopropylbenzene and bromobenzene are used as the inert water-immiscible organic solvents.

7. Process according to claims 1 to 6, characterised in that the organic solution containing crude dimethyl carbonate is treated with 25 to 50% strength by weight aqueous alkali metal hydroxide solution, the alkali metal hydoxide solution is separated off and the organic phase is worked up by distillation.

## Revendications

1. Procédé de fabrication de carbonate de diméthyle par réaction du méthanol avec le phosgène et/ou le chloroformiate de méthyle, caractérisé en ce qu'on exécute la réaction en présence d'une solution aqueuse d'hydroxyde alcalin et en présence de solvants organiques inertes non miscibles avec l'eau à des températures dans l'intervalle de $-20°C$ à $+40°C$.

2. Procédé selon la revendication 1, caractérisé en ce qu'on utilise une solution aqueuse de 15 à 50% en poids d'hydroxyde alcalin.

3. Procédé selon les revendications 1 et 2, caractérisé en ce qu'on utilise par mole de phosgène 2 à 2,6 moles ou, par mole de chloroformiate de méthyle, 1 à 1,3 mole d'hydroxyde alcalin en solution aqueuse.

4. Procédé selon les revendications 1 à 3, caractérisé en ce qu'on utilise comme solution d'hydroxyde alcalin une solution d'hydroxyde de sodium et/ou de potassium.

5. Procédé selon les revendications 1 à 4, caractérisé en ce qu'on utilise comme solvants organiques inertes non miscibles avec l'eau des hydrocarbures aliphatiques et cycloaliphatiques ayant 1 à 30 atomes de carbone, des hydrocarbures aromatiques et araliphatiques ayant 6 à 30 atomes de carbone, des éthers aliphatiques et araliphatiques ayant 5 à 30 atomes de carbone et/ou des amides, nitriles et esters d'acides carboxyliques aliphatiques, araliphatiques at aromatiques ayant 5 à 30 atomes de carbone, qui tous peuvent éventeullement être substitués par du fluor, du chlore ou du brome.

6. Procédé selon les revendications 1 à 5, caractérisé en ce qu'on utilise comme solvants organiques inertes non miscibles avec l'eau du xylène, cumène, chlorobenzène, chloroxylène, dichlorobenzène, diisopylbenzène, bromobenzène.

7. Procédé selon les revendications 1 à 6, caractérisé en ce qu'on traite la solution organique brute contenant le carbonate de diméthyle avec une solution aqueuse à 25–50% en poids d'hydroxyde alcalin, on sépare la solution d'hydroxyde alcalin et l'on traite la phase organique par distillation.

6